(19) Europäisches Patentamt
European Patent Office
Office européen des brevets

(11) Veröffentlichungsnummer: **0 496 332 A1**

# (12) EUROPÄISCHE PATENTANMELDUNG

(21) Anmeldenummer: **92100872.8**

(51) Int. Cl.5: **C07D 499/68**, A61K 31/43

(22) Anmeldetag: **20.01.92**

(30) Priorität: **23.01.91 DE 4102234**

(43) Veröffentlichungstag der Anmeldung: **29.07.92 Patentblatt 92/31**

(84) Benannte Vertragsstaaten: **AT BE CH DE DK ES FR IT LI NL SE**

(71) Anmelder: **ARZNEIMITTELWERK DRESDEN GmbH**
**Meissner Strasse 35**
**O-8122 Radebeul(DE)**

(72) Erfinder: **Ouittschorr, Gisela, Dipl.-Chem.**
**Heinrich-Heine Strasse 21**
**O-3700 Wernigerode(DE)**
Erfinder: **Teubner, Herbert, Dr. rer. nat.**
**Kantstrasse 40**
**O-3700 Wernigerode(DE)**
Erfinder: **Günter, Sabine**
**Veckenstedter Weg 35**
**O-3700 Wernigerode(DE)**
Erfinder: **Oehlmann, Ragna**
**Oehrenfelder Weg 2**
**O-3701 Wernigerode(DE)**
Erfinder: **Veinberg, Grigorij, Dr.**
**Terbatasstrasse I/3, Wohnung 19a**
**26050 Riga(SU)**
Erfinder: **Kononov, Leonid, Dr.**
**Osolziemastrasse 18, Wohnung 264**
**226000 Riga(SU)**
Erfinder: **Lukewitsch, Edmund, Dr.**
**Jerikiustrasse 23, Wohnung 10**
**226059 Riga(SU)**

(74) Vertreter: **Patentanwälte Beetz - Timpe - Siegfried - Schmitt-Fumian- Mayr**
**Steinsdorfstrasse 10**
**W-8000 München 22(DE)**

## (54) Alpha-Aminochinolinoyl-(3)-penicilline, Verfahren zu ihrer Herstellung und ihre Verwendung in antibakteriellen Mitteln.

(57)

Die Erfindung betrifft α-Aminochinolinoyl-(3)-Penicilline der Formel I,

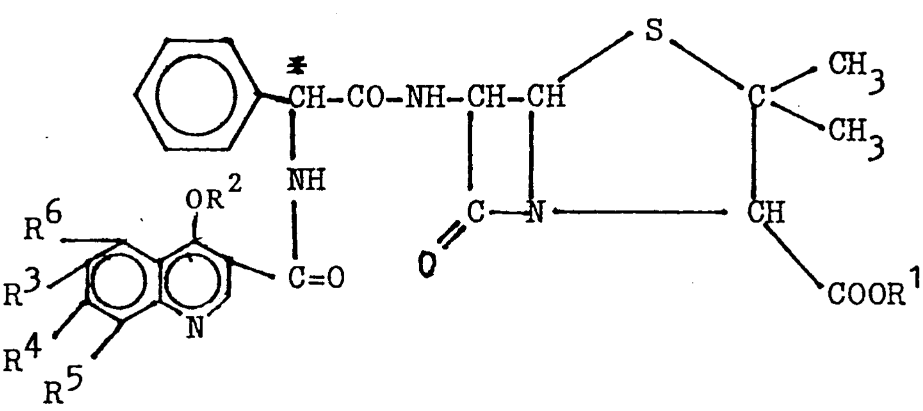

(I),

in der bedeuten:

$R^1$ H, Na, K, Ca, Dialkylammonium, Trialkylammonium;

EP 0 496 332 A1

$R^2$ H, Alkoxycarbonyl;
$R^3$ H, Alkyl, Alkoxy, Halogen;
$R^4$ H, Alkyl, Alkoxy, Halogen, Alkoxymethylen, ggfs. substituiertes Phenoxymethylen, substituiertes Phenylmercaptomethylen, Dialkylaminomethylen, ggfs. in ringgeschlossener Form, wobei $R^3$ und $R^4$ auch ein ankondensiertes Ringsystem bilden können;
$R^5$ H, Alkyl, Alkoxy, Halogen;
$R^6$ H, Alkyl, Alkylcarbonyl,

Verfahren zu ihrer Herstellung durch Umsetzung entsprechender aktivierter Chinolin-3-carbonsäuren mit einem entsprechenden Aminobenzylpenicillin.

Die Verbindungen der Formel I weisen ausgezeichnete antibiotische Wirksamkeit gegenüber grampositiven und gramnegativen Bakterien auf und sind daher als Antibiotika bzw. in antibiotischen Mitteln vorteilhaft einsetzbar.

Die Erfindung betrifft neue α-Aminochinolinoyl-(3)-penicilline der allgemeinen Formel I,

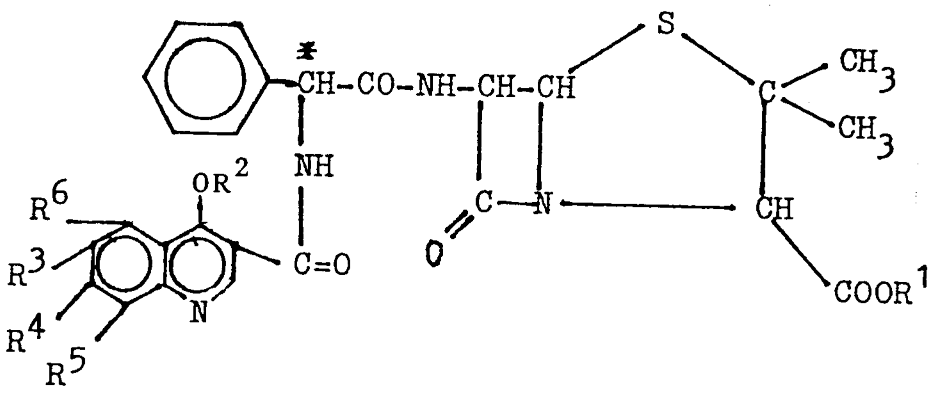

(I),

worin bedeuten:

- $R^1$ H, Na, K, Ca, Dialkylammonium oder Trialkylammonium, mit jeweils geradkettigen und/oder verzweigten $C_{1-6}$- und vorzugsweise $C_{2-4}$-Alkylgruppen,
- $R^2$ H oder Alkoxycarbonyl, vorzugsweise Ethoxycarbonyl oder Isobutoxycarbonyl,
- $R^3$ H, geradkettiges oder verzweigtes $C_{1-6}$-Alkyl, vorzugsweise Ethyl oder Isopropyl, geradkettiges oder verzweigtes $C_{1-5}$-Alkoxy, vorzugsweise Methoxy, oder Halogen, vorzugsweise Brom,
- $R^4$ H, geradkettiges oder verzweigtes Alkyl, vorzugsweise $C_{1-4}$-Alkyl, geradkettiges oder verzweigtes Alkoxy, vorzugsweise $C_{1-6}$-Alkoxy, vorzugsweise Methoxy, Halogen, vorzugsweise Chlor oder Brom, Alkoxymethylen mit geradkettiger oder verzweigter $C_{1-6}$-Alkoxygruppe, Phenoxymethylen, substituiertes Phenoxymethylen, das vorzugsweise in 3- oder 4-Stellung bzw. in 3- und 4-Stellung substituiert ist, bevorzugt mit Alkyl, Alkoxy, Phenoxy, Halogen, vorzugsweise Chlor oder Brom, und/oder mit Acetyl, substituiertes Phenylmercaptomethylen, das vorzugsweise in 4-Stellung mit Alkylgruppen substituiert ist oder Dialkylaminomethylen, das vorzugsweise geradkettige oder verzweigte $C_{1-4}$-Alkylgruppen aufweist oder dessen Alkylgruppen zu Piperidinomethylen, Morpholinomethylen, Pyrrolidinomethylen oder N-Alkylpiperazinomethylen ringgeschlossen sind, wobei die Substituenten $R^3$ und $R^4$ gleich oder verschieden sein können, oder zusammen als $R^3$ und $R^4$ = $(CH)_n$ ein ankondensiertes Ringsystem bilden können, wobei n vorzugsweise 3 oder 4 ist,
- $R^5$ H, geradkettiges oder verzweigtes Alkyl, geradkettiges oder verzweigtes Alkoxy, vorzugsweise Methoxy, oder Halogen, vorzugsweise Chlor,

und

- $R^6$ H, geradkettiges oder verzweigtes Alkyl, vorzugsweise $C_{1-4}$-Alkyl, oder Alkylcarbonyl, vorzugsweise Acetyl oder Propionyl,

Verfahren zu ihrer Herstellung sowie diese Verbindungen enthaltende antibakterielle Mittel.

Die erfindungsgemäßen α-Aminochinolinoyl-(3-)penicilline der allgemeinen Formel I können bezüglich ihres Chiralitätszentrums Č in beiden möglichen R- und S-Konfigurationen sowie als Gemische der daraus resultierenden Diastereomeren vorliegen. Bevorzugt ist die R-Konfiguration bezogen auf das angegebene Chiralitätszentrum Č.

Die Verbindungen der allgemeinen Formel I besitzen gegenüber grampositiven und speziell gegenüber gramnegativen Bakterien eine sehr hohe antibakterielle Wirksamkeit im Vergleich zu eingeführten Acylureidopenicillinen, die als Standards im In-vitro-Test parallel untersucht wurden. Hervorzuheben ist die ausgezeichnete Wirkung gegen die Species Pseudomonas aeruginosa, Klebsiella pneumoniae, Serratia marcescens, Staphylococcus aureus und Staphylococcus epidermidis sowie isoliertes Klinikmaterial von Enterokokken, Escherichia coli, Enterobacter cloacae und Citrobacter freudii.

Einige erfindungsgemäße α-Aminochinolinoyl-(3)-penicilline weisen ferner eine gute Wirkung gegen Lactamase bildende Stämme vom Typ Acetobacter chroococcum, Enterobacter cloacae und Staphylococcus aureus auf.

Die Verbindungen der allgemeinen Formel I können aufgrund ihrer hervorragenden antibakteriellen Wirksamkeit als Arzneimittel bzw. in Arzneimitteln in der Human- und Veterinärmedizin Anwendung finden, insbesondere zur Behandlung von bakteriellen Erkrankungen.

Vom Grundkörper der halbsynthetischen Penicilline, der 6-Aminopenicillansäure, leiten sich eine Vielzahl von Verbindungen ab, die sich durch die Substitution in 6-Stellung an der Aminogruppe unterscheiden.

Diese Substitution bestimmt nicht nur die physikalisch-chemischen Eigenschaften der Penicilline, sondern auch das Wirkungsprofil und die Stabilität gegen inaktivierende Enzyme. Zu den wichtigsten Vertretern gehören das Ampicillin, bei dem der Acylrest in 6-Stellung sich von der D-(-)-α-Aminophenylessigsäure ableitet, das Amoxicillin aus der D-(-)-α-Amino-4-hydroxyphenylessigsäure, das Carbenicillin aus der α-Carboxyphenylessigsäure, ein speziell gegen Pseudomonas wirksames Penicillin und die ß-lactamasestabilen Penicilline vom Oxacillintyp, die durch Acylierung von 6-Aminopenicillansäure mit einer aktivierten Form von substituierten Isoxazolcarbonsäuren hergestellt werden können. Diese aufgeführten Penicilline zeichnen sich durch besondere Wirksamkeit gegen gramnegative Keime aus, wobei teilweise Lactamasestabilität vorliegt. Durch die vorhandenen Lücken im Wirkungsspektrum ist eine weitere Suche nach neuen, gut wirksamen α-Aminoacylpenicillinen nötig. Durch Derivatisierung der funktionellen Gruppen dieser Wirkstoffe, speziell der α-Aminogruppe des Ampicillins bzw. des Amoxicillins, wurden deren Eigenschaften verbessert.

Aus der Patentliteratur sind eine große Anzahl von Veröffentlichungen bekannt, die diese Wirkstoffverbesserung zum Ziel haben, wobei speziell durch Acylierung der α-Aminogruppe über eine Carbonylgruppe ein heterocyclisches Ringsystem in die Seitenkette eingeführt wird. Hierzu zählen besonders DE 2152967, DE 2152968, DE 2226822, DE 2258973, DE 2104579, DE 2104580, BE 767648, DD 117882, US 4112090, JP 8218686 und JP 8218687.

Die als Acylkomponenten eingesetzten Heterocyclen kommen in aktivierter Form zum Einsatz, d.h. als Säurehalogenide, vorzugsweise Säurechloride, als Säureazide, als gemischte Anhydride, darstellbar durch Umsetzung der heterocyclischen Carbonsäuren mit Chlorkohlensäureestern, vorzugsweise Chlorkohlensäureethyl- oder Chlorkohlensäureisobutylester, oder als aktivierte Ester, die durch Umsetzung mit Chlorkohlensäure-5-norbornen-2,3-dioximidester bzw. N,N'-Bis(5-norbornen-2,3-dicarboximidoyl)-carbonat synthetisiert werden können.

Ferner sind aus der Patentliteratur substituierte Chinolin-3-carbonsäureester, die als Chinolone in die Arzneimittelpalette eingegangen sind, mit guten antibakteriellen Eigenschaften bekannt, z.B. aus DE 3142854 und BE 870576.

Der Erfindung liegt die Aufgabe zugrunde, neue α-Aminochinolinoyl-(3)-penicilline der allgemeinen Formel I zu synthetisieren, um mit dieser Substanzklasse neue halbsynthetische Penicilline, die gegen grampositive und speziell gegen gramnegative Bakterienspecies hochwirksam sind und parallel dazu teilweise eine gute Wirkung gegen β-Lactamase bildende Stämme aufweisen, zu erhalten. Dabei soll eine gute Möglichkeit eröffnet werden, dem Resistenzverhalten, mit dem in wiederholten Passagen stets erneut zu rechnen ist, entgegenzuwirken.

Die guten antibakteriellen Eigenschaften von substituierten Chinolin-3-carbonsäure, bekannt durch die Klasse der Chinolone, sollen erfindungsgemäß mit den bekannten, gut antibakteriell wirksamen Penicillinen, z.B. Ampicillin, in Einklang gebracht werden, um das Wirkungsspektrum dadurch wesentlich zu erweitern. Ferner sollen Verfahren zur Herstellung dieser Verbindungen und diese Verbindungen enthaltende pharmazeutische Mittel angegeben werden.

Die Aufgabe wird durch die unabhängigen Ansprüche gelöst. Die abhängigen Ansprüche betreffen vorteilhafte Ausführungsformen der Erfindungskonzeption.

Die erfindungsgemäßen α-Aminochinolinoyl-(3)-penicilline besitzen die oben angegebene Formel I mit den dort angeführten Bedeutungen der Substituenten.

Die Herstellung der α-Aminochinolinoyl-(3)-penicilline der Formel I erfolgt erfindungsgemäß insbesondere durch Umsetzung von substituierten Chinolin-3-carbonsäuren der allgemeinen Formel II,

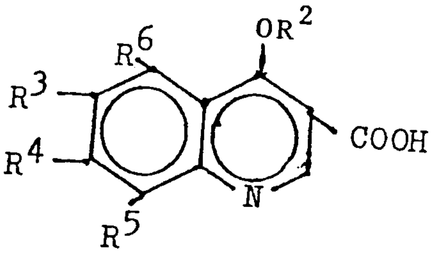

(II),

worin $R^2$, $R^3$, $R^4$, $R^5$ und $R^6$ die oben definierte Bedeutung besitzen, in aktivierter Form mit einem Aminobenzylpenicillin der allgemeinen Formel III,

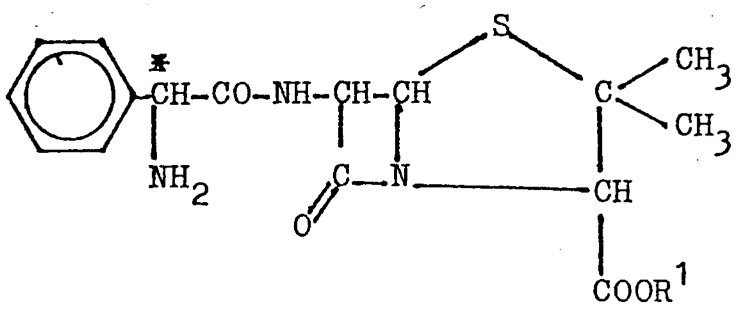

(III),

in der $R^1$ die oben definierte Bedeutung aufweist.

Die Verbindung der Formel II wird entsprechend in einer aktivierten Form eingesetzt. Sie kann z.B. als gemischtes Anhydrid eingesetzt werden, das insbesondere durch Umsetzung der Carboxylgruppe mit Chlorkohlensäureestern, vorzugsweise Chlorkohlensäureethylester oder Chlorkohlensäureisobutylester, hergestellt werden kann. Die Verbindung der Formel II kann auch in Form von aktivierten Estern eingesetzt werden, die durch Umsetzung der Verbindung der Formel II insbesondere mit Chlorkohlensäure-5-norbornen-2,3-dicarboximidester oder N,N'-Bis(5-norbornen-2,3-dicarboximidoyl)-carbonat erhältlich sind.

Bei der Darstellung der gemischten Anhydride, ausgehend von den entsprechenden substituierten Chinolin-3-carbonsäuren der allgemeinen Formel II durch Umsetzung mit Chlorkohlensäureethylester bzw. Chlorkohlensäureisobutylester werden die in 4-Stellung alkoxycarbonylsubstituierten Chinolin-3-carbonsäurederivate erhalten, worin $R^2$ der allgemeinen Formel II die unter Formel I angegebene Bedeutung aufweist. Dieser Syntheseschritt bei der Bildung des gemischten Anhydrids in 4-Stellung der substituierten Chinolincarbonsäuren der allgemeinen Formel II ist irreversibel, was durch NMR-Spektroskopie der erhaltenen α-Aminochinolinoyl-(3-)penicilline der allgemeinen Formel I nachgewiesen wurde.

Die Umsetzung der Verbindungen der Formel II mit entsprechenden Chlorkohlensäurealkylestern wird vorteilhaft in einem wasserfreien organischen Lösungsmittel, vorzugsweise Chlorkohlenwasserstoffen, wie Dichlormethan, cyclischen Ethern, z.B. Tetrahydrofuran oder Dioxan, oder Dimethylformamid, durchgeführt, wobei günstigerweise bei Temperaturen im Bereich von -40 bis 0 °C und vorzugsweise im Bereich von -20 bis -10 °C verfahren wird. Bei dieser Umsetzung wird ferner günstigerweise in Gegenwart von sekundären Aminen gearbeitet, vorzugsweise Triethylamin.

Die Herstellung aktivierter Ester aus den Verbindungen der Formel II durch Umsetzung mit Chlorkohlensäure-5-norbornen-2,3-dioximidesteroder N,N'-Bis(5-norbornen-2,3-dicarboximidoyl)-carbonat wird vorzugsweise in einem wasserfreien organischen Lösungsmittel, vorzugsweise Chlorkohlenwasserstoffen, wie Dichlormethan, cyclischen Ethern, wie Tetrahydrofuran oder Dioxan, oder Dimethylformamid, durchgeführt. Die Reaktionstemperatur liegt bei dieser Umsetzung vorteilhaft im Bereich von -40 bis +10 °C und noch günstiger im Bereich von -20 bis -5 °C. Auch diese Reaktion wird günstigerweise in Gegenwart eines sekundären Amins, vorzugsweise Triethylamin, vorgenommen.

Die Aminobenzylpenicilline der Formel III werden bei der Umsetzung mit den Verbindungen der Formel II vorteilhaft in wasserfreier Form als Salze, vorzugsweise in Form der Natrium-, Kalium- oder Trialkylammoniumsalze, eingesetzt. Die Alkylgruppen solcher Trialkylammoniumsalze weisen vorteilhaft 2 bis 4 C-Atome auf. Die Verbindungen der Formel III können ferner auch in Form geeigneter Ester, insbesondere in Form von Trialkylsilylestern mit $C_{1-4}$-Alkylgruppen, eingesetzt werden.

Die Umsetzung der Verbindungen der Formel II mit den Verbindungen der Formel III, d.h. die Acylierungsreaktion zur Herstellung der Verbindungen der Formel I, wird vorteilhaft in wasserfreien organischen Lösungsmitteln, wie z.B. Chlorkohlenwasserstoffen, vorzugsweise Dichlormethan, cyclischen Ethern, vorzugsweise Tetrahydrofuran oder Dioxan, oder Dimethylformamid, vorgenommen. Die Reaktionstemperatur bei der Acylierung liegt günstigerweise im Bereich von -40 bis +30 °C und noch bevorzugter im Bereich von -20 bis +10 °C.

Die erhaltenen neuen halbsynthetischen α-Aminochinolinoyl-(3)-penicilline der Formel I können in Form der freien Carbonsäuren als auch in Form ihrer Salze isoliert werden. Die Herstellung des Natriumsalzes kann beispielsweise durch Umsetzung der entsprechenden Carbonsäure mit dem Natriumsalz der 2-Ethylhexansäure erfolgen. Das Natriumsalz ist lagerstabil.

Weiterhin konnte überraschend gefunden werden, daß die neuen α-Aminochinolinoyl-(3)-penicilline der allgemeinen Formel I nur dann ausgezeichnete antibakterielle Wirkung aufweisen, wenn beide Ringsysteme des Chinolinoylheterocyclus aromatische Struktur aufweisen und nicht wie bei den Chinolonen am Stickstoffatom substituiert sind. Daraus ist abzuleiten, daß aus der Kombination zweier gut antibakteriell

wirksamer Komponenten, in diesem Falle Aminobenzylpenicilline und Chinolone, nicht automatisch ein Reaktionsprodukt resultiert, das ebenfalls gute antibakterielle Eigenschaften aufweisen muß, sondern daß es ganz exakt auf die Substitution am Chinolinringsystem für die Erzielung dieses Effektes ankommt.

Die durch unterschiedliche Acylierung gewonnenen neuen α-Aminochinolinoyl-(3)-penicilline der allgemeinen Formel I sind speziell gegen gramnegative Keime, z.B. Pseudomonas aeruginosa, Klebsiella pneumoniae, Serratia marcescens, Staphylococcus aureus und Staphylococcus epidermidis, sowie gegen Enterokokken, Escherichia coli, Enterobacter cloacae und Citrobacter freudii hochwirksam. Ein Teil von ihnen weist eine gute Wirkung gegen Lactamase bildende Stämme, wie Acetobacter chroococcum, Enterobacter cloacae und Staphylococcus aureus auf.

Die antibiotische Wirksamkeit erfindungsgemäßer Verbindungen wurde in der üblichen Weise durch Ermittlung der Hemmkonzentrationen nach Verdünnung ermittelt.

Die erhaltenen Ergebnisse sind in der nachstehenden Tabelle zusammengefaßt.

**Tabellarische Übersicht bei In-vitro-Tests erhaltenen Ergebnissen**

| | I | II | III | IV | V | VI | VII | VIII | IX | X | XI | XII | XIII | XIV |
|---|---|---|---|---|---|---|---|---|---|---|---|---|---|---|
| Beisp. 2 | 7 | 8 | 9 | 6 | 5 | 8 | 9 | 8 | 4 | 5 | 5 | 8 | 6 | 6 |
| Beisp. 3 | 8 | 9 | 10 | 7 | 5 | 7 | 9 | 9 | 3 | 5 | 4 | 8 | 6 | 6 |
| Beisp. 4 | 10 | 9 | 9 | 8 | 7 | 8 | 8 | 8 | 5 | 7 | 6 | 8 | 7 | 6 |
| Beisp. 5 | 9 | 8 | 9 | 9 | 7 | 7 | 7 | 8 | 6 | 7 | 6 | 8 | 6 | 6 |
| Beisp. 6 | 8 | 8 | 10 | 9 | 6 | 8 | 9 | 8 | 5 | 6 | 5 | 8 | 7 | 7 |
| Beisp. 9 | 8 | 8 | 10 | 10 | 4 | 8 | 8 | 8 | 5 | 6 | 4 | 7 | 4 | 4 |
| Beisp. 11 | 7 | 7 | 9 | 8 | 5 | 7 | 7 | 7 | 4 | 5 | 5 | 7 | 5 | 5 |
| Beisp. 12 | 7 | 9 | 10 | 7 | 5 | 7 | 9 | 7 | 6 | 7 | 5 | 7 | 6 | 6 |
| Beisp. 14 | 6 | 9 | 11 | 9 | 6 | 8 | 9 | 9 | 7 | 7 | 6 | 9 | 7 | 7 |
| Beisp. 15 | 7 | 9 | 10 | 7 | 7 | 8 | 9 | 9 | 6 | 7 | 6 | 8 | 7 | 7 |
| Beisp. 17 | 8 | 8 | 9 | 6 | 6 | 7 | 10 | 8 | 4 | 6 | 4 | 8 | 6 | 6 |
| Beisp. 18 | 8 | 8 | 10 | 9 | 6 | 7 | 9 | 7 | 10 | 6 | 4 | 8 | 6 | 6 |
| Beisp. 20 | 8 | 8 | 10 | 10 | 5 | 7 | 8 | 6 | 4 | 6 | 5 | 7 | 5 | 5 |
| Beisp. 21 | 8 | 8 | 10 | 9 | 6 | 7 | 9 | 8 | 5 | 6 | 5 | 8 | 6 | 6 |
| Beisp. 22 | 8 | 8 | 10 | 9 | 5 | 8 | 9 | 8 | 4 | 5 | 5 | 8 | 6 | 7 |
| Vergleichssubstanz Ampicillin | 11 | 9 | 5 | 1 | -1 | 9 | 5 | 3 | 5 | 6 | 6 | 5 | -1 | -1 |

Bezeichnung des verwendeten Stammaterials (I bis XIV)

| | | |
|---|---|---|
| I | = Bacillus subtilis | SG 119 |
| II | = Staphylococcus aureus | SG 511 |
| III | = Proteus vulgaris | OX 19 |
| IV | = Pseudomonas aeruginosa | NCTC 10701 |
| V | = Pseudomonas aeruginosa | SG 137 |
| VI | = Salmonella gallinarium | ATCC 9184 |
| VII | = Klebsiella pneumoniae | SG 117 |
| VIII | = Serratia marcescens | SG 621 |
| IX | = Staphylococcus aureus | Laborstämme |
| X | = Staphylococcus epidermidis | Laborstämme |
| XI | = Enterokokken | Laborstämme |
| XII | = Escherichia coli | Laborstämme |
| XIII | = Enterobacter cloacae | Laborstämme |
| XIV | = Citrobacter freudii | Laborstämme |

Erläuterung der Verdünnungsreihe (-1 bis 14)

| | | |
|---|---|---|
| -1 | = Wachstum bei 0,2 mM nicht gehemmt | |
| 1 | = bei 0,2 mM | kein sichtbares Wachstum vorhanden |
| 2 | = bei 0,1 mM | kein sichtbares Wachstum vorhanden |
| 3 | = bei 0,05 mM | kein sichtbares Wachstum vorhanden |
| 4 | = bei 0,025 mM | kein sichtbares Wachstum vorhanden |
| 5 | = bei 0,0125 mM | kein sichtbares Wachstum vorhanden |
| 6 | = bei 0,00625 mM | kein sichtbares Wachstum vorhanden |
| 7 | = bei 0,00312 mM | kein sichtbares Wachstum vorhanden |
| 8 | = bei 0,00156 mM | kein sichtbares Wachstum vorhanden |
| 9 | = bei 0,00078 mM | kein sichtbares Wachstum vorhanden |
| 10 | = bei 0,00039 mM | kein sichtbares Wachstum vorhanden |
| 11 | = bei 0,0002 mM | kein sichtbares Wachstum vorhanden |
| 12 | = bei 0,0001 mM | kein sichtbares Wachstum vorhanden |
| 13 | = bei 0,00005 mM | kein sichtbares Wachstum vorhanden |
| 14 | = bei 0,000025 mM | kein sichtbares Wachstum vorhanden |

Aus der tabellarischen Übersicht geht hervor, daß die neuen α-Aminochinolinoyl-(3)-penicilline der allgemeinen Formel I, die speziell in $R^4$-Stellung einen ringgeschlossenen Morpholinomethylen-, einen Acetylphenoxymethylen- oder einen Alkoxymethylenrest mit 1 bis 4 C-Atomen, der geradkettige oder verzweigte Alkylgruppen aufweist, bzw. ein ankondensiertes Ringsystem in $R^5$- und $R^3$-Stellung tragen, die beste antibakterielle Wirkung, speziell gegen die oben aufgeführten Bakterienspecies, aufweisen.

Die erfindungsgemäßen pharmazeutischen Mittel enthalten eine oder mehrere Verbindungen der allgemeinen Formel I bzw. entsprechende, pharmazeutisch geeignete Salze. Nach einer bevorzugten Ausführungsform enthalten entsprechende Zusammensetzungen 0,0002 bis 0,025 mmol Wirkstoff in Form einer oder mehrerer Verbindungen der Formel I. Die erfindungsgemäßen pharmazeutischen Zusammensetzungen

können ferner auch weitere, bekannte Wirkstoffe enthalten.

Die erfindungsgemäßen Verbindungen lassen sich in üblicher Weise galenisch formulieren, wobei herkömmliche Excipientien verwendet werden können. Auch hinsichtlich der Darreichungsformen bestehen im Rahmen der Erfindung keine Beschränkungen, da die erfindungsgemäßen Verbindungen beispielsweise insbesondere oral wie auch parenteral verabreicht werden können. Entsprechende Formulierungen sind dem Fachmann geläufig.

Die nachfolgenden Ausführungsbeispiele erläutern die Herstellung erfindungsgemäßer Verbindungen.

**Beispiel 1**

6-[D-(-)-(α-([4'-Ethoxycarbonyloxy-6'-ethyl-7'-(4''-chlor)-phenoxymethylen-chinolinoyl-3']-carboxamido)-α-phenylacetamido]-penicillansäure-Natriumsalz

2,55 g (0,0067 mol) Natriumsalz der 4-Hydroxy-7-(4'-chlorphenoxymethylen)-6-ethylchinolin-3-carbonsäure werden in 50 ml wasserfreiem Tetrahydrofuran suspendiert; die Lösung wird auf -20 °C gekühlt. Es werden 1,2 ml (0,014 mol) Chlorkohlensäureethylester zugetropft, wonach 30 min im Eisbad nachgerührt wird. Daraufhin wird eine Lösung von 2,5 g (0,0067 mol) Ampicillin-Natrium in einem Gemisch aus 15 ml Wasser und 15 ml Tetrahydrofuran abgekühlt und zu der Reaktionsmischung unter Kühlen zugegeben. Das Eisbad wird entfernt und der Ansatz nochmals 30 min nachgerührt. Dann wird im Vakuum eingedampft und der Rückstand in Wasser gelöst. Nach der Zugabe von Essigsäureethylester wird mit verdünnter Salzsäure angesäuert, und die Phasen werden getrennt. Es wird mit Essigsäureethylester nachextrahiert; die organischen Phasen werden gewaschen, getrocknet und erneut im Vakuum eingedampft. Der Rückstand wird in wenig getrocknetem Methylenchlorid gelöst und zu einer Lösung von 0,0067 mol Natriumethylhexanoat-(2) in getrocknetem Methylenchlorid unter Kühlung zugetropft. Das Natriumsalz der oben genannten Penicillansäure wird evtl. durch Zugabe von getrocknetem Ether oder Petrolether ausgefällt. Anschließend wird abgesaugt und mit Petrolether gewaschen. Es werden 2,5 g $\hat{=}$ 51,8 % d.Th. des oben genannten Endprodukts erhalten. Dies zeigt bei 1780 $cm^{-1}$ im IR-Spektrum die β-Lactambande.

**Beispiel 2**

6-[D-(-)-α-([4'-Ethoxycarbonyloxy-6'-ethyl-7'-(4''-brom)-phenoxymethylen-chinolinoyl-3']-carboxamido)-α-phenylacetamido]-penicillansäure-Natriumsalz

2,1 g (0,005 mol) Natriumsalz der 4-Hydroxy-7-(4'-bromphenoxymethylen)-6-ethyl-chinolin-3-carbonsäure werden analog Beispiel 1 umgesetzt. Man erhält 2,5 g $\hat{=}$ 65,2 % d.Th. des oben genannten Natriumsalzes mit einer β-Lactambande bei 1775 $cm^{-1}$ im IR-Spektrum.

**Beispiel 3**

6-[D-(-)-α-([4'-Ethoxycarbonyloxy-6'-ethyl-7'-(4''-methyl)-phenoxymethylen-chinolinoyl-3']-carboxamido)-α-phenylacetamido]-penicillansäure-Natriumsalz

1,8 g (0,005 mol) Natriumsalz der 4-Hydroxy-7-(4'-methyl-phenoxymethylen)-6-ethyl-chinolin-3-carbonsäure werden analog Beispiel 1 umgesetzt. Man erhält 2,1 g $\hat{=}$ 61,1 % d.Th. des oben genannten Natriumsalzes. Es zeigt die charakteristische β-Lactambande bei 1780 $cm^{-1}$ im IR-Spektrum.

**Beispiel 4**

6-[D-(-)-α-([4'-Ethoxycarbonyloxy-6'-ethyl-7'-n-butoxymethylen-chinolinoyl-3']-carboxamido)-α-phenylacetamido]-penicillansäure-Natriumsalz

1,04 g (0,003 mol) 4-Hydroxy-7n-butoxymethylen-6-ethyl-chinolin-3-carbonsäure werden unter Zugabe von 1,26 ml (0,009 mol) Triethylamin in 50 ml getrocknetem Methylenchlorid gelöst; die Lösung wird auf -20 °C gekühlt. Nach Zugabe von 0,54 ml (0,006 mol) Chlorkohlensäureethylester wird 30 min im Eisbad nachgerührt. Dann wird eine Lösung von 1,26 g (0,0036 mol) wasserfreiem Ampicillin in 20 ml getrocknetem Methylenchlorid und 1,26 ml (0,009 mol) Triethylamin zugetropft, wobei die Temperatur nicht über -10 °C ansteigen sollte. Das Reaktionsgemisch wird 30 min im Eisbad und 30 min bei Raumtemperatur nachgerührt. Daraufhin wird es im Vakuum eingedampft; der Rückstand wird in Wasser gelöst. Nach der Zugabe

von Essigsäureethylester wird mit verdünnter Salzsäure angesäuert. Die Phasen werden getrennt. Es wird nachextrahiert, und die organischen Extrakte werden gewaschen, getrocknet und erneut im Vakuum eingedampft. Der Rückstand wird in wenig getrocknetem Methylenchlorid gelöst. Mit 0,003 mol Natriumethylhexanoat wird unter Kühlung mit Eiswasser das Natriumsalz gebildet. Das Salz wird gegebenenfalls mit getrocknetem Ether oder Petrolether gefällt, abgesaugt und mit Petrolether gewaschen.

Man erhält 1,97 g $\hat{=}$ 89,95 % d. Th. des oben genannten Natriumsalzes mit der β-Lactambande im IR-Spektrum bei 1785 $cm^{-1}$.

**Beispiel 5**

6-[D-(-)-α-([4'-Ethoxycarbonyloxy-6'-ethyl-7'-n-propoxymethylen-chinolinoyl-3']-carboxamido)-α-phenylacetamido]-penicillansäure-Natriumsalz

0,87 g (0,003 mol) 4-Hydroxy-7n-propoxymethylen-6-ethyl-chinolin-3-carbonsäure werden analog Beispiel 4 umgesetzt. Man erhält 1,85 g $\hat{=}$ 86,45 % d.Th. des oben genannten Natriumsalzes. Es zeigt die charakteristische β-Lactambande bei 1780 $cm^{-1}$ im IR-Spektrum.

**Beispiel 6**

6-[D-(-)-α-([4'-Ethoxycarbonyloxy-6'-ethyl-7'-ethoxy-methylen-chinolinoyl-3']-carboxamido)-α-phenylacetamido]-penicillansäure-Natriumsalz

0,83 g (0,003 mol) 4-Hydroxy-6-ethyl-7-ethoxymethylenchinolin-3-carbonsäure in 50 ml destilliertem Dimethylformamid werden analog Beispiel 4 umgesetzt. Man erhält 1,35 g $\hat{=}$ 64,3 % d.Th. des oben genannten Natriumsalzes mit der β-Lactambande bei 1780 $cm^{-1}$ im IR-Spektrum.

**Beispiel 7**

6-[D-(-)-α-([4'-Isobutoxycarbonyloxy-8'-ethylchinolinoyl-3']-carboxamido)-α-phenylacetamido]-penicillansäure

0,65 g (0,003 mol) 4-Hydroxy-8-ethylchinolin-3-carbonsäure werden in 50 ml destilliertem Dimethylformamid gelöst, mit 1,25 ml (0,009 mol) Triethylamin versetzt und auf -20 °C gekühlt. Nach der Zugabe von 0,78 ml (0,006 mol) Chlorkohlensäureisobutylester wird 15 min auf -20 °C nachgerührt und daraufhin eine Lösung von 1,26 g (0,0036 mol) wasserfreiem Ampicillin in 25 ml getrocknetem Methylenchlorid und 1,26 ml (0,009 mol) Triethylamin zugetropft. Anschließend wird 30 min im Eisbad und 60 min bei Raumtemperatur nachgerührt. Dann wird im Vakuum weitgehend eingedampft und in viel Wasser aufgenommen. Es wird von etwas Ungelöstem abgesaugt und unter kräftigem Rühren und Kühlen im Eisbad mit verdünnter Salzsäure angesäuert, wobei die Penicillansäure ausfällt. Sie wird abgesaugt, mit Wasser gewaschen und getrocknet. Man erhält 1,4 g $\hat{=}$ 73,4 g der oben genannten Penicillansäure. Sie zeigt die β-Lactambande bei 1785 $cm^{-1}$ im IR-Spektrum.

**Beispiel 8**

6-[D-(-)-α-([4'-Isobutoxycarbonyloxy-7',8'-dimethylchinolinoyl-3']-carboxamido)-α-phenylacetamido]-penicillansäure

0,65 g (0,003 mol) 4-Hydroxy-7,8-dimethylchinolin-3-carbonsäure werden analog Beispiel 7 umgesetzt. Es werden 1,3 g $\hat{=}$ 70,1 % d.Th. der oben genannten Penicillansäure erhalten. Sie zeigt die charakteristische Bande für β-Lactam bei 1785 $cm^{-1}$ im IR-Spektrum.

**Beispiel 9**

6-[D-(-)-α-([4'-Hydroxy-6'-methoxy-chinolinoyl-3']-carboxamido)-α-phenylacetamido]-penicillansäure-Natriumsalz

0,88 g (0,004 mol) 4-Hydroxy-6-methoxychinolin-3-carbonsäure werden in 40 ml getrocknetem Tetrahydrofuran suspendiert und mit 0,54 ml (0,004 mol) Triethylamin versetzt. Das Reaktionsgemisch wird während der Salzbildung mit Eiswasser gekühlt. Dann werden eine Lösung von 0,97 g (0,004 mol)

Chlorkohlensäure-5-norbornen-2,3-dioximidester in 10 ml getrocknetem Methylenchlorid und eine Spatelspitze Dimethylaminopyridin zugegeben; der Ansatz wird 1 1/2 h bei Raumtemperatur umgesetzt. Es wird eine Lösung von 1,68 g (0,0048 mol) wasserfreiem Ampicillin in 20 ml getrocknetem Methylenchlorid mit 1,89 ml (0,0133 mol) Triethylamin hergestellt, die auf -17 °C gekühlt und mit 0,55 ml (0,0048 mol) Trimethylchlorsilan versetzt wird. Sie wird 30 min im Eisbad nachgerührt; daraufhin wird das oben erhaltene Reaktionsgemisch zugegeben. Nach einer weiteren Nachreaktionszeit von 30 min im Eisbad und 30 min bei Raumtemperatur werden 5 ml Wasser zugegeben, und es wird weitere 10 min gerührt. Das organische Lösungsmittel wird im Vakuum weitgehend entfernt und der Rückstand mit Wasser versetzt. Es wird mit Essigsäureethylester versetzt und mit verdünnter Salzsäure angesäuert. Die Phasen werden getrennt, und die wäßrige Phase wird noch zweimal mit Essigsäureethylester nachextrahiert. Die vereinigten organischen Phasen werden mit gesättigter Kochsalzlösung sorgfältig gewaschen, über Natriumsulfat getrocknet und erneut im Vakuum eingedampft. Die resultierende Penicillansäure wird in wenig getrocknetem Methylenchlorid gelöst. Mit der äquivalenten Menge Natrium-2-ethylhexanoat wird unter Kühlung mit Eiswasser das Natriumsalz gebildet. Es wird erforderlichenfalls mit getrocknetem Ether oder Petrolether gefällt, abgesaugt und getrocknet.

1,99 g $\widehat{=}$ 74,2 % d.Th. des oben genannten Natriumsalzes werden gewonnen, das die typische β-Lactambande bei 1780 $cm^{-1}$ im IR-Spektrum aufweist.

**Beispiel 10**

6-[D-(-)-α-([4'-Hydroxy-6'-bromchinolinoyl-3']-carboxamido)-α-phenylacetamido]-penicillansäure-Natriumsalz

1,07 g (0,004 mol) 4-Hydroxy-6-bromchinolin-3-carbonsäure werden analog Beispiel 9 umgesetzt. Dabei werden 2,0 g $\widehat{=}$ 69,4 % d.Th. des oben genannten Natriumsalzes mit einer β-Lactambande bei 1775 $cm^{-1}$ im IR-Spektrum erhalten.

**Beispiel 11**

6-[D-(-)-α-([4'-Isobutoxycarbonyloxy-6'-isopropyl-chinolinoyl-3']-carboxamido)-α-phenylacetamido]-penicillansäure-Natriumsalz

0,69 g (0,003 mol) 4-Hydroxy-6-isopropylchinolin-3-carbonsäure werden in 50 ml absolutem Tetrahydrofuran mit 0,84 ml (0,006 mol) Triethylamin versetzt und auf -20 °C gekühlt. Nach Zugabe von 0,78 ml (0,006 mol) Chlorkohlensäureisobutylester wird 30 min bei -20 °C nachreagieren gelassen. Daraufhin wird eine Lösung von 1,26 g (0,0036 mol) wasserfreiem Ampicillin in 25 ml getrocknetem Methylenchlorid und 1,26 ml (0,009 mol) Triethylamin bei mindestens -10 °C zugetropft. Der Ansatz wird 30 min im Eisbad und 30 min bei Raumtemperatur gehalten. Dann wird das Reaktionsgemisch im Vakuum eingedampft, der Rückstand in Wasser aufgenommen, evtl. von Ungelöstem abfiltriert, mit Essigsäureethylester versetzt und mit verdünnter Salzsäure angesäuert. Die Phasen werden getrennt; die wäßrige Phase wird zweimal mit Essigsäureethylester nachextrahiert. Die vereinigten organischen Phasen werden gewaschen, getrocknet und erneut im Vakuum eingeengt. Der Rückstand wird in wenig getrocknetem Methylenchlorid gelöst. Mit 0,003 mol Natrium-2-ethylhexanoat wird das Natriumsalz gebildet.

Man erhält 1,63 g $\widehat{=}$ 79,5 % d.Th. des oben genannten Natriumsalzes, das die charakteristische β-Lactambande bei 1780 $cm^{-1}$ im IR-Spektrum aufweist.

**Beispiel 12**

6-[D-(-)-α-([4'-Isobutoxycarbonyloxybenzo[1,2n]chinolinoyl-3']-carboxamido)-α-phenylacetamido]-penicillansäure-Natriumsalz

0,72 g (0,003 mol) 4-Hydroxy-3-carbonsäure-benzo[1,2n]-chinolin werden analog Beispiel 11 umgesetzt, wobei zum Lösen der Chinolincarbonsäure 70 ml destilliertes Dimethylformamid verwendet werden. Es resultieren 1,06 g $\widehat{=}$ 51,0 % d.Th. des oben genannten Natriumsalzes mit der β-Lactambande bei 1780 $cm^{-1}$ im IR-Spektrum.

**Beispiel 13**

6-[D-(-)-α-([4'-Isobutoxycarbonyloxy-5'-propionyl-chinolinoyl-3']-carboxamido)-α-phenylacetamido]-penicillansäure-Natriumsalz

0,69 g (0,003 mol) 4-Hydroxy-5-propionylchinolin-3-carbonsäure werden analog Beispiel 11 umgesetzt, wobei zum Lösen der Chinolincarbonsäure 50 ml getrocknetes Methylenchlorid Verwendung finden. Man erhält 1,2 g ≙ 57,4 % d.Th. des oben genannten Natriumsalzes mit der β-Lactambande bei 1780 $cm^{-1}$ im IR-Spektrum.

**Beispiel 14**

6-[D-(-)-α-([4'-Isobutoxycarbonyloxy-6'-ethyl-7'-morpholinomethylen-chinolinoyl-3']-carboxamido)-α-phenylacetamido]-penicillansäure-Natriumsalz

0,95 g (0,003 mol) 4-Hydroxy-6-ethyl-7-morpholinomethylen-chinolin-3-carbonsäure werden analog Beispiel 11 umgesetzt, wobei zum Lösen der substituierten Chinolin-3-carbonsäure 50 ml getrocknetes Methylenchlorid verwendet werden.

1,2 g ≙ 52,6 % d.Th. des oben genannten Natriumsalzes werden erhalten. Es zeigt die charakteristische Bande für β-Lactam im IR-Spektrum bei 1770 $cm^{-1}$.

**Beispiel 15**

6-[D-(-)-α-([4'-Isobutoxycarbonyloxy-6'-ethyl-7'-(4''-acetyl)-phenoxymethylen-chinolinoyl-3']-carboxamido)-α-phenylacetamido]-penicillansäure-Natriumsalz

1,1 g (0,003 mol) 4-Hydroxy-6-ethyl-7-(4'-acetyl)-phenoxymethylen-chinolin-3-carbonsäure werden analog Beispiel 11 umgesetzt, wobei zum Lösen der Chinolincarbonsäure 50 ml destilliertes Dimethylformamid verwendet werden. Es werden 1,7 g ≙ 69,9 % d.Th. des oben genannten Natriumsalzes mit der β-Lactambande bei 1780 $cm^{-1}$ im IR-Spektrum gewonnen.

**Beispiel 16**

6-[D-(-)-α-([4'-Isobutoxycaarbonyloxy-6'-ethyl-7'-(4''-methyl)-phenylmercaptomethylen-chinolinoyl-3']-carboxamido)-α-phenylacetamido]-penicillansäure-Natriumsalz

1,06 g (0,003 mol) 4-Hydroxy-6-ethyl-7-(4'-methyl)-phenylmercapto-methylen-chinolin-3-carbonsäure werden analog Beispiel 11 umgesetzt, wobei zum Lösen der substituierten Chinolin-3-carbonsäure 70 ml destilliertes Dimethylformamid eingesetzt werden. Dabei werden 1,28 g ≙ 54,4 % d.Th. des oben genannten Natriumsalzes erhalten, das die β-Lactambande bei 1780 $cm^{-1}$ im IR-Spektrum aufweist.

**Beispiel 17**

6-[D-(-)-α-([4'-Isobutoxycarbonyloxy-6'-ethyl-7'-(3''-methyl-4''-chlor)-phenoxymethylen-chinolinoyl-3']-carboxamido)-α-phenylacetamido]-penicillansäure-Natriumsalz

1,2 g (0,003 mol) 4-Hydroxy-6-ethyl-7-(3'-methyl-4'-chlor)-phenoxymethylen-chinolin-3-carbonsäure werden analog Beispiel 11 umgesetzt, wobei 1,6 g ≙ 66,6 % d.Th. des oben genannten Natriumsalzes mit der β-Lactambande bei 1785 $cm^{-1}$ erhalten werden.

**Beispiel 18**

6-[D-(-)-α-([4'-Isobutoxycarbonyloxy-6'-ethyl-7'-methoxymethylen-chinolinoyl-3']-carboxamido)-α-phenylacetamido]-penicillansäure-Natriumsalz

0,78 g (0,003 mol) 4-Hydroxy-6-ethyl-7-methoxymethylen-chinolin-3-carbonsäure werden analog Beispiel 11 umgesetzt, wobei zum Lösen der Chinolincarbonsäure 70 ml destilliertes Dimethylformamid eingesetzt werden. Es werden 1,43 g ≙ 66 8 % d.Th. des oben genannten Natriumsalzes erhalten, das im IR-Spektrum die charakteristische β-Lactambande bei 1780 $cm^{-1}$ zeigt.

**Beispiel 19**

6-[D-(-)-α-([4'-Isobutoxycarbonyloxy-8'-methoxychinolinoyl-3']-carboxamido)-α-phenylacetamido]-penicillansäure-Natriumsalz

0,66 g (0,003 mol) 4-Hydroxy-8-methoxychinolin-3-carbonsäure werden analog Beispiel 11 umgesetzt. Dabei werden 2,48 g $\hat{=}$ 73,8 % d.Th. des oben genannten Natriumsalzes gewonnen mit einer β-Lactambande bei 1770 $cm^{-1}$ im IR-Spektrum.

**Beispiel 20**

6-[D-(-)-α-([4'-Isobutoxycarbonyloxy-6',7'-dimethoxychinolinoyl-3']-carboxamido)-α-phenylacetamido]-penicillansäure-Natriumsalz

0,75 g (0,003 mol) 4-Hydroxy-6,7-dimethoxychinolin-3-carbonsäure werden analog Beispiel 11 umgesetzt, wobei 50 ml destilliertes Dimethylformamid zum Lösen der Chinolincarbonsäure verwendet werden. 1,26 g $\hat{=}$ 59,7 % d.Th. des oben genannten Natriumsalzes werden erhalten. Es zeigt im IR-Spektrum die für β-Lactamantibiotika typische Bande bei 1780 $cm^{-1}$.

**Beispiel 21**

6-[D-(-)-α-([4'-Isobutoxycarbonyloxy-6'-ethyl-7'-isopropoxymethylen-chinolinoyl-3']-carboxamido)-α-phenylacetamido]-penicillansäure-Natriumsalz

0,87 g (0,003 mol) 4-Hydroxy-6-ethyl-7-isopropoxymethylenchinolin-3-carbonsäure werden analog Beispiel 11 umgesetzt, wobei zum Lösen der substituierten Chinolin-3-carbonsäure ein Gemisch aus jeweils 20 ml absolutem Tetrahydrofuran, getrocknetem Methylenchlorid und destilliertem Dimethylformamid zum Einsatz gelangt. Es werden 1,42 g $\hat{=}$ 63,9 % d.Th. des oben genannten Natriumsalzes mit der β-Lactambande im IR-Spektrum bei 1785 $cm^{-1}$ erhalten.

**Beispiel 22**

6-[D-(-)-α-([4'-Isobutoxycarbonyloxy-6'-ethyl-7'-s-butoxymethylen-chinolinoyl-3']-carboxamido)-α-phenylacetamido]-penicillansäure-Natriumsalz

0,91 g (0,003 mol) 4-Hydroxy-6-ethyl-7-s-butoxymethylenchinolin-3-carbonsäure werden analog Beispiel 11 in dem in Beispiel 21 angegebenen Lösungsmittelgemisch umgesetzt. Dabei werden 1,49 g $\hat{=}$ 65,6 % d.Th. des oben genannten Natriumsalzes gewonnen, das im IR-Spektrum bei 1780 $cm^{-1}$ die β-Lactambande aufweist.

**Beispiel 23**

6-[D-(-)-α-([4'-Isobutoxycarbonyloxy-6'-ethyl-7'-isobutoxymethylen-chinolinoyl-3']-carboxamido)-α-phenylacetamido]-penicillansäure-Natriumsalz

0,91 g (0,003 mol) 4-Hydroxy-6-ethyl-7-isobutoxymethylenchinolin-3-carbonsäure werden analog Beispiel 11 umgesetzt, wobei als Lösungsmittel ein Gemisch aus gleichen Teilen trockenem Tetrahydrofuran, getrocknetem Methylenchlorid und destilliertem Dimethylformamid eingesetzt werden kann. Es werden 1,75 g $\hat{=}$ 79,9 % d.Th. des Natriumsalzes erhalten; das aufgenommene IR-Spektrum weist bei 1785 $cm^{-1}$ die β-Lactambande auf.

**Beispiel 24**

6-[D-(-)-α-([4'-Isobutoxycarbonyloxy-8'-methoxychinolinyl-3']-carboxamido)-α-phenylacetamido]-penicillansäure-Natriumsalz

0,72 g (0,003 mol) 4-Hydroxy-8-methoxychinolin-3-carbonsäure werden analog Beispiel 11 in 50 ml abs. Tetrahydrofuran umgesetzt. Es werden 1,49 g $\hat{=}$ 73,8 % d.Th. des oben genannten Natriumsalzes erhalten, das im IR-Spektrum bei 1785 $cm^{-1}$ die β-Lactambande zeigt.

**Beispiel 25**

6-[D-(-)-α-([4'-Isobutoxycarbonyloxy-7',8'-dichlorchinolinoyl-3']-carboxamido)-α-phenylacetamido]-penicillansäure-Natriumsalz

0,78 g (0,003 mol) 4-Hydroxy-7,8-dichlorchinolin-3-carbonsäure werden analog Beispiel 11 umgesetzt, wobei als Lösungsmittel 50 ml dest. Dimethylformamid Verwendung finden. Dabei werden 1,07 g ≙ 50,0 % d.Th. des oben genannten Natriumsalzes gewonnen. Es zeigt im IR-Spektrum die typische β-Lactambande bei 1775 $cm^{-1}$.

## Patentansprüche

**1.** α-Aminochinolinoyl-(3)-penicilline der allgemeinen Formel I,

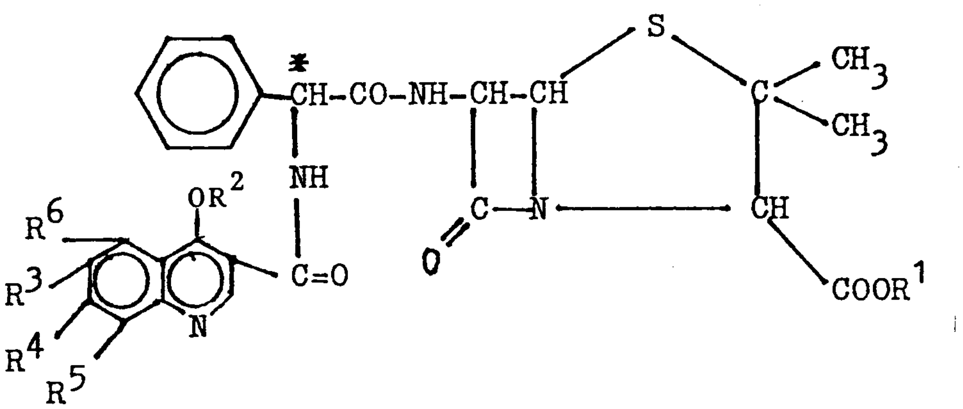

(I),

worin bedeuten:

- $R^1$ H, Na, K, Ca, Dialkylammonium oder Trialkylammonium, mit jeweils geradkettigen und/oder verzweigten $C_{1-6}$- und vorzugsweise $C_{2-4}$-Alkylgruppen,
- $R^2$ H oder Alkoxycarbonyl, vorzugsweise Ethoxycarbonyl oder Isobutoxycarbonyl,
- $R^3$ H, geradkettiges oder verzweigtes $C_{1-6}$-Alkyl, vorzugsweise Ethyl oder Isopropyl, geradkettiges oder verzweigtes $C_{1-5}$-Alkoxy, vorzugsweise Methoxy, oder Halogen, vorzugsweise Brom,
- $R^4$ H, geradkettiges oder verzweigtes Alkyl, vorzugsweise $C_{1-4}$-Alkyl, geradkettiges oder verzweigtes Alkoxy, vorzugsweise $C_{1-6}$-Alkoxy, vorzugsweise Methoxy, Halogen, vorzugsweise Chlor oder Brom, Alkoxymethylen mit geradkettiger oder verzweigter $C_{1-6}$-Alkoxygruppe, Phenoxymethylen, substituiertes Phenoxymethylen, das vorzugsweise in 3- oder 4-Stellung bzw. in 3- und 4-Stellung substituiert ist, bevorzugt mit Alkyl, Alkoxy, Phenoxy, Halogen, vorzugsweise Chlor oder Brom, und/oder mit Acetyl, substituiertes Phenylmercaptomethylen, das vorzugsweise in 4-Stellung mit Alkylgruppen substituiert ist oder Dialkylaminomethylen, das vorzugsweise geradkettige oder verzweigte $C_{1-4}$-Alkylgruppen aufweist oder dessen Alkylgruppen zu Piperidinomethylen, Morpholinomethylen, Pyrrolidinomethylen oder N-Alkylpiperazinomethylen ringgeschlossen sind, wobei die Substituenten $R^3$ und $R^4$ gleich oder verschieden sein können, oder zusammen als $R^3$ und $R^4$ = $(CH)_n$ ein ankondensiertes Ringsystem bilden können, wobei n vorzugsweise 3 oder 4 ist,
- $R^5$ H, geradkettiges oder verzweigtes Alkyl, geradkettiges oder verzweigtes Alkoxy, vorzugsweise Methoxy, oder Halogen, vorzugsweise Chlor,

und

- $R^6$ H, geradkettiges oder verzweigtes Alkyl, vorzugsweise $C_{1-4}$-Alkyl, oder Alkylcarbonyl, vorzugsweise Acetyl oder Propionyl.

**2.** Folgende Verbindungen der Formel I nach Anspruch 1:

6-[D-(-)-α-([4'-Ethoxycarbonyloxy-6'-ethyl-7'-(4''-chlor)-phenoxymethylen-chinolinoyl-3']-carboxamido)-α-phenylacetamido]-penicillansäure-Natriumsalz;

6-[D-(-)-α-([4'-Ethoxycarbonyloxy-6'-ethyl-7'-(4''-brom)-phenoxymethylen-chinolinoyl-3']-carboxamido)-α-phenylacetamido]-penicillansäure-Natriumsalz;

6-[D-(-)-α-([4'-Ethoxycarbonyloxy-6'-ethyl-7'-(4''-methyl)-phenoxymethylen-chinolinoyl-3']-carboxamido)-α-phenylacetamido]-penicillansäure-Natriumsalz;

6-[D-(-)-α-([4'-Ethoxycarbonyloxy-6'-ethyl-7'-n-butoxymethylen-chinolinoyl-3']-carboxamido)-α-phenylacetamido]-penicillansäure-Natriumsalz;

6-[D-(-)-α-([4'-Ethoxycarbonyloxy-6'-ethyl-7'-n-propoxymethylen-chinolinoyl-3']-carboxamido)-α-phenylacetamido]-penicillansäure-Natriumsalz;

6-[D-(-)-α-([4'-Ethoxycarbonyloxy-6'-ethyl-7'-ethoxymethylen-chinolinoyl-3']-carboxamido)-α-phenylacetamido]-penicillansäure-Natriumsalz;

6-[D-(-)-α-([4'-Isobutoxycarbonyloxy-8'-ethyl-chinolinoyl-3']-carboxamido)-α-phenylacetamido]-penicillansäure;

6-[D-(-)-α-([4'-Isobutoxycarbonyloxy-7',8'-dimethyl-chinolinoyl-3']-carboxamido)-α-phenylacetamido]-penicillansäure;

6-[D-(-)-α-([4'-Hydroxy-6'-methoxy-chinolinoyl-3']-carboxamido)-α-phenylacetamido]-penicillansäure-Natriumsalz ;

6-[D-(-)-α-([4'-Hydroxy-6'-brom-chinolinoyl-3']-carboxamido)-α-phenylacetamido]-penicillansäure-Natriumsalz ;

6-[D-(-)-α-([4'-Isobutoxycarbonyloxy-6'-isopropyl-chinolinoyl-3']-carboxamido)-α-phenylacetamido]-penicillansäure-Natriumsalz;

6-[D-(-)-α-([4'-Isobutoxycarbonyloxy-benzo/1,2n/chinolinoyl-3']-carboxamido)-α-phenylacetamido]-penicillansäure-Natriumsalz ;

6-[D-(-)-α-([4'-Isobutoxycarbonyloxy-5'-propionyl-chinolinoyl-3']-carboxamido)-α-phenylacetamido]-penicillansäure-Natriumsalz ;

6-[D-(-)-α-([4'-Isobutoxycarbonyloxy-6'-ethyl-7'-morpholinomethylen-chinolinoyl-3']-carboxamido)-α-phenylacetamido]-penicillansäure-Natriumsalz;

6-[D-(-)-α-([4'-Isobutoxycarbonyloxy-6'-ethyl-7'-(4''-acetyl)-phenoxymethylen-chinolinoyl-3']-carboxamido)-α-phenylacetamido]-penicillansäure-Natriumsalz ;

6-[D-(-)-α-([4'-Isobutoxycarbonyloxy-6'-ethyl-7'-(4''-methyl)-phenylmercaptomethylen-chinolinoyl-3']-carboxamido)-α-phenylacetamido]-penicillansäure-Natriumsalz;

6-[D-(-)-α-([4'-Isobutoxycarbonyloxy-6'-ethyl-7'-(3''-methyl-4''-chlor)-phenoxymethylen-chinolinoyl-3']-carboxamido)-α-phenylacetamido]-penicillansäure-Natriumsalz;

6-[D-(-)-α-([4'-Isobutoxycarbonyloxy-6'-ethyl-7'-methoxymethylen-chinolinoyl-3']-carboxamido)-α-phenylacetamido]-penicillansäure-Natriumsalz ;

6-[D-(-)-α-([4'-Isobutoxycarbonyloxy-8'-methoxy-chinolinoyl3']-carboxamido)-α-phenylacetamido]-penicillansäure-Natriumsalz;

6-[D-(-)-α-([4'-Isobutoxycarbonyloxy-6',7'-dimethoxy-chinolinoyl-3']-carboxamido)-α-phenylacetamido]-penicillansäure-Natriumsalz;

6-[D-(-)-α-([4'-Isobutoxycarbonyloxy-6'-ethyl-7'-isopropoxymethylen-chinolinoyl-3'-]-carboxamido)-α-phenylacetamido]-penicillansäure-Natriumsalz;

6-[D-(-)-α-([4'-Isobutoxycarbonyloxy-6'-ethyl-7'-sek.butoxymethylen-chinolinoyl-3']-carboxamido-α-phenylacetamido]-penicillansäure-Natriumsalz;

6-[D-(-)-α-([4'-Isobutoxycarbonyloxy-6'-ethyl-7'-iso-butoxycillansäure-Natriumsalz;

6-[D-(-)-α-([4'-Isobutoxycarbonyloxy-8'-methoxy-chinolinoyl-3']-carboxamido)-α-phenylacetamido]-penicillansäure-Natriumsalz;

6-[D-(-)-α-([4'-Isobutoxycarbonyloxy-7',8'-dichlor-chinolinoyl-3']-carboxamido)-α-phenylacetamido]-penicillansäure-Natriumsalz.

3. Verfahren zur Herstellung der α-Aminochinolinoyl-(3)-penicilline der allgemeinen Formel I nach den Ansprüchen 1 und 2,

**gekennzeichnet durch**

Umsetzung von substituierten Chinolin-3-carbonsäure der allgemeinen Formel II,

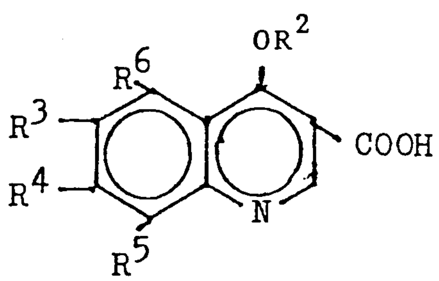

(II),

worin $R^2$ $R^3$, $R^4$, $R^5$ und $R^6$ die in Anspruch 1 definierte Bedeutung besitzen,

in aktivierter Form

mit einem Aminobencylpenicillin der allgemeinen Formel III,

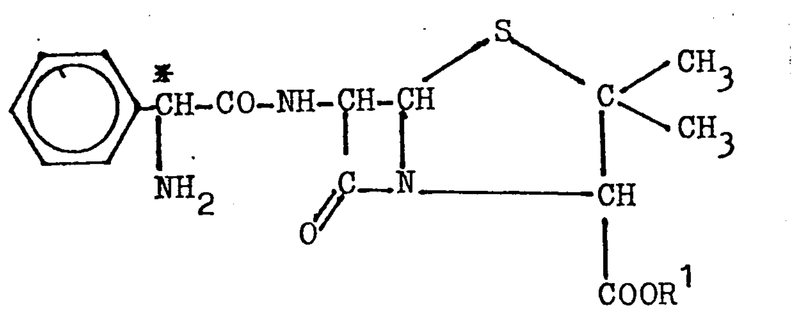

(III),

in der $R^1$ die in Anspruch 1 definierte Bedeutung aufweist.

4. Verfahren nach Anspruch 3, dadurch gekennzeichnet, daß die substituierten Chinolin-3-carbonsäuren der Formel II in aktivierter Form als gemischtes Anhydrid, das durch Umsetzung der Verbindung der Formel II mit Chlorkohlensäureestern, bevorzugt Chlorkohlensäureethylester oder Chlorkohlensäureisobutylester, hergestellt ist, oder als aktivierter Ester, der durch Umsetzung der Verbindung der Formel II mit Chlorkohlensäure-5-norbornen-2,3-dioximidester bzw. N,N'-Bis(5-norbornen-2,3-dicarboximidoyl)-carbonat hergestellt ist, eingesetzt wird.

**5.** Verfahren nach den Ansprüchen 3 oder 4, dadurch gekennzeichnet, daß die Umsetzung der Verbindung der Formel II mit den entsprechenden Chlorkohlensäurealkylestern in einem wasserfreien organischen Lösungsmittel, vorzugsweise Chlorkohlenwasserstoffen, wie Dichlormethan, cyclischen Ethern, wie Tetrahydrofuran oder Dioxan, oder Dimethylformamid, bei Temperaturen im Bereich von -40 bis 0 °C und bevorzugter im Bereich von -20 bis -10 °C in Gegenwart von sekundären Aminen, vorzugsweise Triethylamin, durchgeführt wird.

**6.** Verfahren nach einem der Ansprüche 3 bis 5, dadurch gekennzeichnet, daß die Herstellung der aktivierten Ester aus den Verbindungen der Formel II durch Umsetzung mit chlorkohlensäure-5-norbornen-2,3-dioximidester oder N,N'-Bis(5-norbornen-2,3-dicarboximidoyl)-carbonat in einem wasserfreien organischen Lösungsmittel, vorzugsweise Chlorkohlenwasserstoffen, wie Dichlormethan, cyclischen Ethern, wie Tetrahydrofuran oder Dioxan, oder Dimethylformamid, bevorzugt bei Temperaturen im Bereich von -40 bis =10 °C und noch bevorzugter im Bereich von -20 bis -5 °C, in Gegenwart eines sekundären Amins, vorzugsweise Triethylamin, durchgeführt wird.

**7.** Verfahren nach einem der Ansprüche 3 bis 6, dadurch gekennzeichnet, daß die Verbindungen der Formel III in wasserfreier Form als Salze, vorzugsweise als Natrium-, Kalium- oder Trialkylammoniumssalze mit $C_{2-4}$-Alkylgruppen, oder in Form von Trialkylsilylestern mit $C_{1-4}$-Alkylgruppen eingesetzt werden.

**8.** Verfahren nach einem der Ansprüche 3 bis 7, dadurch gekennzeichnet, daß die Acylierungsreaktion durch Umsetzung der Verbindungen der Formel II mit den Verbindungen der Formel III in wasserfreien organischen Lösungsmitteln, wie Chlorkohlenwasserstoffen, vorzugsweise Dichlormethan, cyclischen Ethern, vorzugsweise Tetrahydrofuran oder Dioxan, oder Dimethylformamid, bei einer Reaktionstemperatur im Bereich von -40 bis +30 °C und noch bevorzugter im Bereich von -20 bis +10 °C durchgeführt wird.

**9.** Pharmazeutische Mittel, dadurch gekennzeichnet, daß sie eine oder mehrere Verbindungen der Formel I nach Anspruch 1 oder 2 enthalten.

**10.** Pharmazeutische Mittel nach Anspruch 9, gekennzeichnet durch einen Gehalt von 0,0002 bis 0,025 mmol einer oder mehrerer Verbindungen der Formel I nach Anspruch 1 oder 2.

Europäisches Patentamt

# EUROPÄISCHER RECHERCHENBERICHT

Nummer der Anmeldung

EP 92 10 0872

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | DE-A-2 165 462 (SUMITOMO CHEMICAL CO. LTD.) * Ansprüche * | 1-10 | C07D499/68 A61K31/43 |
| X | DE-A-2 416 449 (SUMITOMO CHEMICAL CO. LTD.) * Ansprüche * | 1-10 | |
| A | EP-A-0 062 328 (EISAI CO. LTD.) * Ansprüche * | 1-10 | |

RECHERCHIERTE SACHGEBIETE (Int. Cl.5)

C07D
A61K

Der vorliegende Recherchenbericht wurde für alle Patentansprüche erstellt

| Recherchenort | Abschlußdatum der Recherche | Prüfer |
|---|---|---|
| DEN HAAG | 14 APRIL 1992 | CHOULY J. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)